# EUROPÄISCHE PATENTSCHRIFT

(11) **EP 1 827 465 B1**
(45) Veröffentlichungstag und Bekanntmachung des Hinweises auf die Patenterteilung: **19.08.2009**
(21) Anmeldenummer: 04804959.7
(22) Anmeldetag: 21.12.2004
(51) Int. Cl.: A61K 35/64, A61P 17/02

(54) **PRÄPARAT AUS DIPTEREN-PUPPEN ZUR BEHANDLUNG VON WUNDEN**
PREPARATION MADE FROM DIPTERA LARVAE FOR THE TREATMENT OF WOUNDS
PREPARATIONS A BASE DE CHRYSALIDES DE DIPTERES DESTINEES A TRAITER DES BLESSURES

(43) Veröffentlichungstag der Anmeldung: 05.09.2007
(73) Patentinhaber: Alpha-Biocare GmbH, 40225 Düsseldorf (DE)
(72) Erfinder: D'HAESE, Jochen, 42551 Velbert (DE); MEHLHORN, Heinz, 41468 Neuss (DE); Prof. Dr. RUZICKA, Thomas Derma.-Klinik der LMU München, 80337 München (DE); SCHMIDT, Jürgen, 40221 Düsseldorf (DE); STEGE, Helger, 79111 Freiburg (DE)
(74) Vertreter: Gille Hrabal Struck Neidlein Prop Roos
(86) Internationale Anmeldenummer: PCT/EP2004/053624
(87) Internationale Veröffentlichungsnummer: WO 2006/066619

(56) Entgegenhaltungen:
- DE-A1- 10 327 489
- DATABASE WPI Section Ch, Week 199433 Derwent Publications Ltd., London, GB; Class B04, AN 1994-269454 XP002326900 & JP 06 199898 A (SANSEI SEIYAKU KK) 19. Juli 1994 (1994-07-19)
- DATABASE WPI Section Ch, Week 200008 Derwent Publications Ltd., London, GB; Class B04, AN 2000-087779 XP002326901 & CN 1 231 131 A (SHANGHAI WILD RESOURCE HIGH SCI & TECHNO) 13. Oktober 1999 (1999-10-13)
- HORN K L ET AL: "MAGGOT THERAPY FOR SUBACUTE MASTOIDITIS" Juni 1976 (1976-06), ARCHIVES OF OTOLARYNGOLOGY, AMERICAN MEDICAL ASSOCIATION, CHICAGO, IL, US, PAGE(S) 377-379 , XP009022002 ISSN: 0003-9977 das ganze Dokument

## Beschreibung

Die Erfindung betrifft die Verwendung von Extrakten oder Isolaten, erhältlich aus Puppen von einzelnen Arten der Fliegengattungen Sarcophaga, Musca, Lucilia, Phormia, oder Calliphora oder erhältlich aus zwei oder mehr Arten dieser Gattungen, zur Herstellung eines Präparats zur Behandlung von Wunden.

Bei der Wundheilung von Mensch und Tier spielen eine Vielzahl von Faktoren in einer Kaskade von Prozessen eine entscheidende Rolle. Der unmittelbare Verlauf lässt sich unabhängig vom Typ der Wunden (chronisch/akut) in drei unterschiedlich lange Hauptphasen untergliedern. Es beginnt mit der sogenannten exsudativen Phase, die von der proliferativen und schließlich von der Reparationsphase gefolgt wird.

In der exsudativen Phase, die klinisch durch ein Wundoödem und Wundschmerz charakterisiert ist, stehen Reaktionen mit vasokonstriktorischen und hämostaseologischen Effekten im Vordergrund. Entstandene Gefäßdefekte werden durch Thrombozyten verschlossen. Die dabei ablaufenden Prozesse locken Makrophagen, neutrophile Granulozyten und Lymphozyten an, wodurch Phagozytose - Prozesse initiiert werden, die letztlich das Gewebedebridement einleiten.

Nach der Beseitigung von Zelltrümmern wird mit der Einwanderung von Fibroblasten und Gefößendothelzellen die proliferative Phase der Wundheilung begonnen. Neben dem auf Einwanderung begründeten Anwachsen der Zellmasse erfolgt die verstärkte Freisetzung von Zytokinen und auch Wachstumsfaktoren, was wiederum die Zellproliferation und auch weitere Gefäßneubildungen fördert. Während gleichzeitigem Matrixumbau in dieser Phase (Umwandlung von Kollagen I in Typ F) entsteht letztlich ein gut kapillarisiertes Granulationsgewebe, das zahlreiche Makrophagen, Fibroblasten und auch Mastzellen einschließt. Auf diese Phase folgt dann die Epithelisierungs- und Reparationsphase. Diese ist gekennzeichnet durch eine Einwanderung von peripheren Keratinozyten in die Wunde und deren Wundkontraktion. Während jetzt die Kapillarendichte reduziert wird, nimmt der Kollagengehalt noch weiter zu, was für die Festigkeit der Narbe von großer Bedeutung ist.

Kommt es aber zu Störungen im Ablauf der komplexen Prozesse dieser drei Wundheilungsphasen, kann eine z.T. beträchtliche Verzögerung der Wundheilung die Folge sein. Wird dieser Vorgang auf 6-8 Wochen ausgedehnt, so gilt dies als eine chronische Wundheilungsstörung. Derartiges tritt häufig als Folge unterschiedlicher Erkrankungen auf (z.B. als Folge von Immundepression und Diabetes mellitus, als Ergebnis einer Varikose oder bestimmten Superinfektionen etc.). Maßnahmen zur Verbesserung der Wundheilung zielen dann auf die Beschleunigung der Abläufe in den drei erwähnten Phasen der Wundheilung. So muss insbesondere eine der Hauptursachen der verzögerten Wundheilung - die mangelnde Bildung von Granulationsgewebe - beseitigt werden, die entweder auf vermindertem endogenen Wunddebridement oder aber auf der übermäßigen Bildung von Zelldetritus beruht.

Als Maßnahmen zur Reinigung nekrotisch oder fibrinös belegter akuter oder chronischer Wunden eignen sich chirurgische Verfahren, wie eine Küretage oder die Wundexzision. Allerdings werden durch diese Verfahren erneut Traumata gesetzt, die, ganz besonders bei chronischen Wunden, zu einer erneuten Verzögerung der Wundheilung führen können. Bei infizierten Wunden sind diese Maßnahmen nicht oder nur unter einer Antibiotikaprophylaxe einsetzbar. Ein weiteres Problem stellt die Schmerzhaftigkeit des Eingriffs dar, der nur unter einer Lokalanästhesie oder unter einer Vollnarkose durchgeführt werden kann. Bei größeren debridierenden chirurgischen Maßnahmen ist eine postoperative Immobilisierung erforderlich. Diese ist ganz besonders bei älteren Patienten, die unter zahlreichen weiteren Erkrankungen leiden, häufig unerwünscht. Ein semichirurgisches Verfahren, das ein gewisses technisches Verständnis auch seitens der Patienten voraussetzt, ist die Vakuumversiegelung. Hierbei werden unter permanentem Sog die Wunden okklusiv abgedeckt und die Beläge kontinuierlich durch den Unterdruck abgetragen. Dieses Verfahren ist sehr effektiv, aber erfordert in der Regel auch eine Immobilisierung. Rein pharmakologische Verfahren basieren auf dem Einsatz von proteolytischen Enzymen. Bei den meisten auf dem Markt befindlichen Substanzen handelt es sich entweder um Enzyme aus Einzellern oder diese Produkte sind bovinen Ursprungs. Eine Wirksamkeit in der Praxis findet sich häufig nur gering, da entweder die Konzentration zu niedrig gewählt ist oder die Halbwertszeit der Produkte zu kurz ist oder aber die Zielsubstanzen dem Wirtsspektrum der Enzyme nicht entsprechen. Es ist zu erwähnen, dass aufgrund geringer Wirksamkeit oder einem fehlenden Wirksamkeitsnachweis eine Reihe kommerziell erhältlicher Produkte nicht mehr auf dem Markt angeboten werden.

Als weitere Maßnahme zur Verbesserung der Wundheilung ist auch der Einsatz von lebenden Fliegenmaden der Art Lucilia sericata bekannt.

Diese Art der Therapie, bei der äußerlich gereinigte Maden auf die Wunden verbracht werden, ist aus der Volksmedizin seit Jahrhunderten bekannt. Die Maden (Larven) der Fliegen ernähren sich von nekrotischem Gewebe, nicht aber von gesundem Gewebe, und dringen auch nicht in tiefere Schichten ein. Die Lucilia sericata - Larven beißen oder nagen das nekrotische Gewebe nicht ab, sondern lysieren es offenbar durch per os ausgeschiedene Substanzen und saugen dann den Zellbrei auf. Sie besitzen nämlich keine Mundwerkzeuge, denn die beiden Mundhaken dienen lediglich zur Verankerung in der Haut und können dabei Schmerzen hervorrufen.

Die Probleme beim routinemäßigen Einsatz dieser lebenden Maden in toto bei der Wundheilung liegen in drei Bereichen. Das erste Problem und sicherlich nicht das unwichtigste ist ein ethisches. So ekeln sich nicht wenige Menschen vor den krabbelnden Maden, die zudem noch beim Festhalten in der Wunde mit ihren Mundhaken durchaus beachtliche Schmerzen bereiten. Dann ist es auch schwierig, lebende Fliegenmaden frei von potentiellen Krankheitserregern wie Bakterien oder Pilzen zu züchten, um mikrobielle Infektionen zu vermeiden. Auch beim Transport vom Labor zum Patienten müssen die Fliegenmaden keimfrei gehalten werden. Ein ebenso bedeutsames Problem ergibt sich aber auch aus der Logistik. Fliegenmaden können auf diesem Stadium nicht "arretiert" werden, sondern wachsen weiter und verpuppen sich nach einiger Zeit. Daher ist es nur sehr schwer möglich, entsprechende Larven von geeigneter Größe bei Ankunft eines entsprechenden Patienten in der Klinik einsatzfähig zu haben und für jede neue Applikation bereitzuhalten.

Im Bemühen, neue und besser wirksame Medikamente zur Behandlung von chronischen und akuten Wunden bei Mensch und Tier zu entwickeln, wurde nun überraschenderweise gefunden, dass die erfindungsgemäß verwendeten Extrakte oder Isolate, die Inhaltsstoffe von Puppen von Fliegen der Gattungen Sarcophaga, Musca, Calliphora, Lucilia oder Phormia enthalten, die genannten Nachteile der gegenwärtigen Therapiemaßnahmen bei der Wundheilung beheben können.

Aus den DE 10149153 A1, DE 10138303 A1 sowie DE 19901134 A1 war weiterhin die Verwendung von Extrakten aus Fliegenlarven bzw. Fliegenmaden bekannt. Es war jedoch nicht zu erwarten, dass Puppenextrakte die gleiche Wirkung wie Madenextrakte besitzen, da Puppen nicht mehr fressen, also eigentlich keine lysierenden Enzyme benötigen. Es ist daher überraschend, dass der aus Puppen gewonnene Extrakt überhaupt wirkt. Weiterhin besitzen die Puppenextrakte auch zahlreiche Vorteile gegenüber Fliegenmadenextrakten. So kann der Inhalt der Puppen völlig sauber gewonnen werden, weil die Puppenhülle nach außen dicht ist und die Oberfläche der Puppen sterilisiert werden kann. Weiterhin lässt sich das Puppenstadium durch Lagerung in der Kälte für Wochen oder gar Monate problemlos halten, was eine extreme Erleichterung gegenüber der Verwendung von lebenden Maden darstellt, da sich deren Entwicklung nicht mehr stoppen lässt. Weitere Vorteile der Verwendung von Puppen gegenüber Fliegenmaden sind in der Beschreibung erwähnt. So führt die Anwendung von Fliegenmaden bei den Patienten zu Schmerzen und Kot der Fliegenmaden kann die Wunden verschmutzen und führt zu üblem Geruch. Fliegenmaden führen außerdem zu Ekel bei den Patienten und besitzen somit eine sehr geringe Patienten-Compliance.

Die erfindungsgemäß verwendeten Extrakte oder Isolate aus Fliegenpuppen stellen somit eine signifikante Verbesserung der gegenwärtigen praktizierten Therapie mit lebenden Maden von Fliegen oder der Verwendung von Extrakten daraus dar. Durch Einsatz der Inhaltsstoffe von Puppen ist es nämlich möglich, deren Oberfläche intensivst zu säubern und zu sterilisieren, die gewonnenen Isolate aufzureinigen, steril zu filtrieren, zu lyophilisieren, zu lagern und erst bei Bedarf - dann zudem exakt dosiert - auf sie zurückzugreifen. Als derartiges Fertigpräparat bieten diese so präparierten Inhaltsstoffe kontinuierliche Eingreifmöglichkeiten, die nicht vom jeweiligen Entwicklungszustand lebender Maden abhängen.

Die Erfindung betrifft die Verwendung von Extrakten oder Isolaten, erhältlich aus Puppen von einzelnen Arten der Fliegengattungen Musca, Calliphora, Phormia, Sarcophaga oder Lucilia, oder erhältlich aus Puppen von zwei oder mehr Arten dieser Gattungen, zur Herstellung eines Präparats zur Behandlung von Wunden.

Geeignete Arten aus der Gattung Sarcophaga sind z.B. S. carnaria, aus der Gattung Lucilia: L. sericata, aus Musca: M. domestica, Phormia: P. regina, sowie aus der Gattung Calliphora; C. erythrocephala, Diese Arten, die im Freiland weit verbreitet sind und zudem weltweit in vielen wissenschaftlichen Institutionen gehalten werden, können somit als definiertes Material leicht und in großer Menge für entsprechende Extraktionsprozesse herangezüchtet werden.

Die Erfindung betrifft insbesondere die Verwendung von Extrakten mit wundheilender Wirkung, die aus dem Inneren von Puppen gewonnen werden, sobald deren Kokon ausgebildet ist.

Die erfindungsgemäß verwendeten Extrakte mit wundheilender Wirkung können hergestellt werden, indem die Puppenhülle zunächst äußerlich gereinigt und von potentiellen Erregern befreit worden ist. Dann kann das Puppeninnere entnommen werden, wobei das gewonnene Homogenisat in eine wasserlösliche und eine nicht-lösliche Fraktion aufgetrennt werden kann. Die löslichen Anteile werden aufbewahrt.

Die Homogenisierung kann durch Zusatz von Chemikalien, durch mechanische Homogenisierung oder durch Anwendung von Ultraschall erfolgen. Die Trennung von löslichen und ungelösten Anteilen kann z.B. durch Filtration und/oder Zehtrifugation durchgeführt werden. Das gesamte Verfahren wird zur besseren Konservierung von Wirksubstanzen im Isolat unter Kühlung durchgeführt. Anschließend erfolgt die Aufbewahrung der gelösten Homogenisationsbestandteile durch Einfrieren oder durch Gefriertrocknung. Ferner können weitere bekannte Substanzen (z.B. Puffer, Salze, Antioxidationsmittel oder microbizide Substanzen etc.) zur Stabilisierung der Präparatbestandteile (z.B. Proteine, Enzyme, Proenzyme, Lipide, niedermolekulare Anteile etc.) zugesetzt werden.

Die Herstellung der erfindungsgemäß verwendeten Extrakte oder Isolate erfolgt dadurch, dass Larven der erwähnten Dipteren auf geeigneten Substraten (z.B. Pferdefleisch) herangezüchtet und dann auf Holzspäne gegeben werden, wo sich die Maden verpuppen können. Die Fliegenpuppen werden gesammelt, äußerlich gereinigt und verwendet.

Die erfindungsgemäß hergestellten Präparate schließen im allgemeinen jegliche aus den erwähnten Dipteren-Puppen erhältliche Extrakte oder Isolate daraus, die zur Wundbehandlung geeignet sind, ein.

Die gewonnenen insbesondere wässrigen Extrakte oder Isolate werden vor der Aufarbeitung in geeigneten Trägermedien, z.B. physiologische Salzlösungen, sterile Elektrolytlösungen, Albuminlösungen, Ölen, Fetten etc., aufbewahrt.

Die Erfindung betrifft weiterhin die Verwendung der Extrakte oder Isolate zur Herstellung eines Präparats zur Wundbehandlung, insbesondere zur topischen Anwendung.

Die erfindungsgemäß hergestellten Präparate haben einen wirksamen Gehalt an den Extrakten oder loslaten mit wundheilender Wirkung zusammen mit einem pharmazeutisch geeigneten physiologisch verträglichen Trägerstoff, Zusatzstoff und/oder anderen Wirk- und Hilfsstoffen. Wegen der pharmakologischen Eigenschaften eignen sich die erfindungsgemäß verwendeten Präparate bzw. Arzneimittel insbesondere zur Therapie von oberflächlichen oder tiefen chronischen wie akuten Wunden jeglicher Genese.

Unter dem Begriff "chronische und akute Wunden jeglicher Genese" werden beispielsweise Wunden verstanden wie Operationswunden, die gezielt oder ungewollt sekundär heilen sollen, Schnitt-, Stich-, Schürf-, Biss-, oder Schussverletzungen sowie andere Wunden, die nicht primär mittels chirurgischer Naht oder einem primären Wundverschluss behandelt werden können. Ferner sind mit dem Begriff der akuten Wunden auch alle Wunden gemeint, die durch ein mikrobielle infektion nicht primär abheilen können und alle Wunden, deren Manifestation 4 Wochen und weniger beträgt. Chronische Wunden sind alle Verletzungen, die mit der Aufhebung der Integrität des Epithels einhergehen und länger als 4 Wochen manifest sind. Insbesondere sind hiermit schlecht heilende Wunden auf der Basis eines Diabetes mellitus, einer Varikose oder einer Venenthrombose, eines rheumatischen Leidens, einer Vaskulitis, einer arteriellen Verschlusskrankheit, eines Leidens der Lymphgefäße, hämatologischer Erkrankungen und während oder nach Infektionen der Wunden gemeint.

Die erfindungsgemäß verwendeten Extrakte oder Isolate mit wundheilender Wirkung können mit einem pharmazeutisch geeigneten und physiologisch verträglichen Träger und gegebenenfalls weiteren geeigneten Wirk-, Zusatz- oder Hilfsstoffen in eine geeignete Darreichungsform gebracht werden.

Die Applikation der erfindungsgemäß hergestellten Präparate erfolgt in der Regel topisch. Geeignete pharmazeutische Zusammensetzungen für die topische Anwendung auf der Haut liegen vorzugsweise als Salbe, Lösung, Suspension, Creme, Puder, liposomalen oder olesomalen Formulierungen, Gel, Lotion, Paste, Spray, Aerosol oder Öl vor. Als Träger können Vaseline, Lanolin, Polyethylenglykole, Alkohole und Kombinationen von zwei oder mehreren dieser Substanzen verwendet werden. Die erfindungsgemäß verwendeten Extrakte oder Enzymisolate sind im allgemeinen in einer Konzentration von 0,1 Gew.-% bis 100 Gew.-% der galenischen Zusammensetzung vorhanden, vorzugsweise von 1 ,0 Gew.-% bis 60 Gew.-%.

Auch eine transdermale Verabreichung der Präparate bzw. Arzneimittel ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise die erfindungsgemäß verwendeten Extrakte oder Isolate in einer gegebenenfalls gepufferten wässrigen Lösung, gelöst und/oder dispergiert in einem Haftmittel oder dispergiert in einem Polymer. Eine geeignete Wirkstoff-Konzentration beträgt von etwa 0,1 % Gew.-% bis 75 Gew.-%, vorzugsweise von 1 % Gew.-% bis 70 Gew.-%. Als eine besondere Möglichkeit kann der Wirkstoff, wie beispielsweise in Pharmaceutical Research, 2: 318 (1986) beschrieben, durch Elektrotransport oder lontophorese freigesetzt werden.

Die erfindungsgemäß verwendeten Extrakte oder Isolate aus diesen Extrakten können ferner auch auf die Wunde appliziert werden durch Wundauflagen aus Gaze, aus Alginaten, aus hydrokolloidalen Materialien, Schaumstoffen und Silikonauflagen, die mit diesen Extrakten oder Isolaten beschichtet, getränkt oder behandelt wurden und deshalb in der Lage sind, die Wirkstoffe in oder auf die Wunde oder Wundoberflüche abzugeben.

Geeignete feste oder galenische Zubereitungsformen sind beispielsweise Granulate, Pulver, Dragees, Tabletten, (Mikro-) Kapseln, Suppositorien, Sirupe, Säfte, Lösungen, Suspensionen, Emulsionen, Tropfen oder injizierbare Lösungen sowie Präparate mit protrahierter Wirkstoff-Freigabe, bei deren Herstellung übliche Hilfsmittel oder Trägerstoffe, wie Spreng-, Binde-, Überzugs-, Quellungs-, Gleit- oder Schmiermittel, Geschmackstoffe, Süßungsmittel und Lösungsvermittler, Verwendung finden. Als häufig verwendete Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuss-, oder Sesamöl, Polyethylenglykol und Lösungsmittel wie etwa steriles Wasser und ein- oder mehrwertige Alkohole wie Glycerin genannt.

Ferner können die Extrakte auch in galenischen Zubereitungen eingesetzt werden, die die Wirkstoffe, z.B. Enzyme, in inaktiver Form enthalten und die dann in oder auf die Wunde aufgetragen werden und durch Zugabe von spezifischen Substanzen aktiviert werden.

Ein besonders bevorzugtes Präparat wird durch Herstellen eines wässrigen Extraktes und anschließende Lyophilisation des wässrigen Extraktes, insbesondere des von unlöslichen Anteilen befreiten Überstands des Extraktes erhalten. Auf diese Weise wird ein haltbares und dosierbares Präparat erhalten.

Beispiele sind die Verwendung eines Pulvers oder von Lyophilisaten, die mit physiologischen Lösungen (z.B. 0,9%-iger wässriger NaCl) aufgelöst werden. Bei ausreichender Stabilität kann die galenische Zubereitung auch eine Lösung sein.

Die Applikation der geeigneten pharmazeutischen Präparate erfolgt nach mechanischer Wundreinigung. Die mechanische Wundreinigung erfolgt beispielsweise durch ein Bad oder eine Spülung der Wunde mit Ringerlaktat. Die Wunde wird wahlweise nach Applikation oder erfindungsgemäßen Extrakte oder Isolaten mittels hydrokolloidaler Wunddressings oder mittels selbstklebender Operations-Folie bedeckt. Verbandswechsel mit jeweils neuer Gabe der erfindungsgemäßen Extrakte oder lsolate erfolgen täglich.

### Beispiel A: Herstellung von Extrakten

Larven der Arten Musca domestica, Calliphora erythrocephala, Lucilia sericata, Phormia regina und/oder Sarcophaga carnaria werden von frischem, oberflächlich abgeflammten, und nicht kontaminiertem Pferdefleisch geerntet und in geeigneten Behältern zur Verpuppung gelagert. Die dann gesammelten Puppen werden in mehreren Waschschritten in sterilisierter, isoosmotischer Salzlösung äußerlich gesäubert. Danach werden die Puppen zerdrückt, und der Inhalt wird in einem Trägermedium auf Eis gesammelt. Das Trägermedium kann entweder wässriger oder hydrophober Natur sein. Danach werden die Inhaltsstoffe homogenisiert. Dies geschieht in mehreren Schritten mittels Ultraschall oder mechanischer Homogenisatoren oder durch Zusatz von Lösungsmitteln. Auf eine ständige Kühlung bei etwa 4° C Celsius wird geachtet. Nach Erhalt einer homogenen Flüssigkeit wird der Extrakt durch einen sterilen Filter mit 0,2 µm Porenweite steril filtriert. Im letzten Bearbeitungsschritt wird der Extrakt aliquotiert und in flüssigem Stickstoff eingefroren. Die dauerhafte Lagerung erfolgt bei etwa - 18° C bis -80° C.

### Anwendungsbeispiel A: Wundbehandlung bei Patienten

Ein 83-jähriger Patient mit einem PTS entwickelte ein chronisches Ulcus cruris an typischer Lokalisation. Trotz intensiver Behandlung über mehr als 12 Wochen, einschließlich enzymatischer Wundtherapeutika, antientzündlicher, desinfizierender Maßnahmen und einer suffizienten Kompressionstherapie gelang es nicht, das Ulcus in Abheilung zu bringen. Die Wunde zeigte auch nach 12 Wochen deutliche Beläge, die fibrinösen teils entzündlichen Ursprungs waren. Die Isolate wurden einmal pro Tag in wässriger Form und als Lyophilisat auf die Wunde aufgetragen. Danach wurde die Wunde mit einer wirkstofffreien Fettgaze bedeckt und mittels zweier Kompressionsbinden gewickelt. Innerhalb von 7 Tagen zeigte sich ein stark ausgeprägtes Granulationsgewebe, ohne Reste eines fibrinösen Belages. Die Applikation der Isolate erfolgte weiterhin täglich einmal. Innerhalb von 35 Tagen kam es zu einer mehr als 60%igen Reduktion der Wundfläche und nach 59 Tagen war das Ulcus komplett abgeheilt.

### Beispiel B: Extrakt aus einer Fliegenpuppenart

1500 Fliegenpuppen der Art Lucilia sericata wurden nach und nach aus den Produktionskäfigen entnommen und im Kühlschrank gesammelt, bis die Anzahl erreicht wurde. Dann wurden sie mechanisch homogenisiert und zunächst mit der Kühlzentrifuge für 2 Stunden zentrifugiert. Der Überstand wurde dann noch für 12 Stunden mit einer Beckmann Ultrazentrifuge zentrifugiert. Der Überstand wurde dann sterilisiert und in Portionen von 1 ml bei -80°C tiefgefroren. Zum Einsatz kamen nach dem Auftauen Portionen von 1 ml.

Der gewonnene Extrakt wurde zudem standardgemäß lyophilisiert und kann dann in praxisgemäßen (zum direkten Einsatz) Aliquots bei +4° C oder tieferen Temperaturen im Kühlschrank gelagert werden.

### Beispiel C: Extrakt aus zwei Fliegenpuppenarten

Je 750 Puppen der Fliegenarten Lucilia sericata und Sarcophaga carnaria wurden Kühlschrank gesammelt. Danach wurde mit jeder Gruppe einzeln so verfahren wie oben in Beispiel A beschrieben. Die Lagerung des Extraktes erfolgte für jede Art einzeln. Zur Behandlung wurden dann je 0,5 ml der Extrakte beider Puppenarten aufgetaut und vermischt, dann kräftig durchgeschüttelt und auf die Wunde verbracht.

Die gewonnenen Extrakte wurden zudem standardgemäß lyophilisiert und können dann in praxisgemäßen (zum direkten Einsatz) Aliquots bei +4° C oder tieferen Temperaturen im Kühlschrank gelagert werden.

Anwendungsbeispiel B: Produkt aus Puppen einer Fliegenart

Es stellte sich eine 76-jährige Patientin mit einem seit Jahren rezidivierenden Ulcus cruris bei schwerer chronisch venöser Insuffizienz vor. Das vorhandene Ulcus bestand seit mehreren Monaten und war mit konventionellen Maßnahmen der feuchten Wundbehandlung vorbehandelt. Klinisch präsentierte sich ein fibrinös und gering nekrotisch belegtes Ulkus. Im Rahmen eines Heilversuches wurden Extrakte aus Puppen der Gattung Lucilia sericata (1 ml/2 cm²) auf die Wunde aufgetragen und mit einer wirkstofffreien Fettgaze sowie trockenen Kompressen und einer Kompressionsbinde abgedeckt. Die Anwendung erfolgte initial täglich. Nach 14 Tagen konnte eine fast vollständige Beseitigung der Fibrinbeläge und die Induktion eines Granulationsgewebes beobachtet werden. Im Anschluss an diese Phase der Behandlung erfolgte die Therapie alle 2 Tage. Jetzt wurde das Ulkus zusätzlich zur Applikation mit Extrakten aus Lucilia sericata-Puppen mit feuchten Kompressen oder aber hydrokolloidhaltigen Wunddressings abgedeckt. Nach weiteren vier Wochen beobachteten wir eine initiale Epithelisierung. Nach insgesamt 94 Tagen der Wundbehandlung zeigte sich ein komplett abgeheiltes Ulkus. Der Therapieerfolg lässt sich eindeutig der Wirkung der Extrakte aus Lucilia sericata-Puppen zuordnen, da die übrigen Maßnahmen in der Vorgeschichte nicht zu einem Therapieerfolg geführt haben.

### Anwendungsbeispiel C: Produkt aus Puppen zweier Fliegengattungen

Eine 64-jährige Patientin litt seit Monaten unter einer chronischen Wunde im Bereich des linken Unterschenkels auf Grund einer rezidivierenden Vaskulitis. Die Therapie mit Glukokortikosteroiden systemisch hatte die Aktivität der Gefäßentzündung gebremst, konnte aber nicht zu einer effektiven Induktion von Granulationsgewebe führen. Tägliches chirurgisches Debridement war für die Patientin sehr schmerzhaft und zeigte regelhaft erneutes Auftreten von Fibrinbelägen. Erst unter der täglichen Applikation von Extrakten (50% zu 50% Volumenanteil) aus Puppen der Fliegengattungen Lucilia sericata und Sarcophaga carnaria kam es innerhalb von 10 Tagen zu einer kompletten Beseitigung des nekrotischen und fibrinösen Materials, das die Wunde bedeckt hatte und einer beginnenden Epithelisierung. Somit ist dieser Extrakt aus zwei Gattungen von Fliegen ebenso wirksam. Dies eröffnet Einsparmaßnahmen.

## Patentansprüche

1. Verwendung von Extrakten oder Isolaten, erhältlich aus Puppen von einzelnen Arten der Fliegengattungen Musca, Calliphora, Phormia, Sarcophaga oder Lucilia oder erhältlich aus Puppen von zwei oder mehr Arten dieser Gattungen, zur Herstellung eines Präparats zur Behandlung von Wunden.

2. Verwendung gemäß Anspruch 1 , **dadurch gekennzeichnet, dass** es aus den Arten Musca domestica, Calliphora erythrocephala, Sarcophaga carnaria, Phormia regina, Lucilia sericata und/oder Lucilia caesar stammt.

3. Verwendung gemäß einem oder mehreren der Ansprüche 1 und 2, **dadurch gekennzeichnet, dass** ganze Puppen oder Bestandteile der Puppen der Gattungen Musca, Calliphora, Phormia, Sarcophaga und/oder Lucilia mechanisch, durch Zusatz von Chemikalien und/oder durch Ultraschall homogenisiert werden.

4. Verwendung gemäß der Anspruch 3, **dadurch gekennzeichnet, dass** die Homogenate aufgereinigt werden, z.B. von nicht gelösten Bestandteilen befreit werden, und lösliche Bestandteile konserviert werden.

5. Verwendung gemäß einem oder mehreren der Ansprüche 1 bis 4, **dadurch gekennzeichnet, dass** die Trennung von unlöslichen Bestandteilen durch Zentrifugation, Ultrazentrifugation, Phasentrennung oder Filtration erfolgt und ausschließlich der Überstand verwendet wird.

6. Verwendung gemäß einem oder mehreren der Ansprüche 1 bis 5, **dadurch gekennzeichnet, dass** eine Sterilfiltration mit einem Filter mit einem Porendurchmesser von 0,1 µm bis 0,4 µm erfolgt.

7. Verwendung gemäß einem oder mehreren der Ansprüche 1 bis 6, **dadurch gekennzeichnet, dass** Mittel zur Konservierung, z.B. mikrobizide oder antioxidative Stoffe, zugesetzt werden.

8. Verwendung gemäß einem oder mehreren der Ansprüche 1 bis 7, **gekennzeichnet durch** einen Zusatz von pharmazeutisch geeigneten und physiologisch verträglichen Hilfsstoffen.

## Claims

1. Use of extracts or isolates, obtainable from pupae of individual species of the fly genera Musca, Calliphora, Phormia, Sarcophaga or Lucilia or obtainable from pupae of two or more species of those genera, in the production of a preparation for the treatment of wounds.

2. Use according to claim 1, **characterised in that** it derives from the species Musca domestica, Calliphora erythrocephala, Sarcophaga carnaria, Phormia regina, Lucilia sericata and/or Lucilia caesar.

3. Use according to one or more of claims 1 and 2, **characterised in that** whole pupae or parts of the pupae of the genera Musca, Calliphora, Phormia, Sarcophaga and/or Lucilia are homogenised mechanically, by addition of chemicals and/or by ultrasound.

4. Use according to claim 3, **characterised in that** the homogenates are purified, for example are freed of undissolved constituents, and soluble constituents are retained.

5. Use according to one or more of claims 1 to 4, **characterised in that** the separation of insoluble constituents is carried out by centrifugation, ultracentrifugation, phase separation or filtration and only the supernatant is used.

6. Use according to one or more of claims 1 to 5, **characterised in that** sterile filtration using a filter having a pore diameter of from 0.1 µm to 0.4 µm is carried out.

7. Use according to one or more of claims 1 to 6, **characterised in that** preservatives, for example microbicidal or antioxidant substances, are added.

8. Use according to one or more of claims 1 to 7, **characterised by** the addition of pharmaceutically suitable and physiologically acceptable auxiliary substances.

## Revendications

1. Utilisation d'extraits ou d'isolats, que l'on peut obtenir à partir de chrysalides provenant d'espèces individuelles des genres de mouches Musca, Calliphora, Phormia, Sarcophaga ou Lucilla, ou que l'on peut obtenir à partir de chrysalides de deux ou de plusieurs espèces de ces genres, dans le but de fabriquer une préparation permettant le traitement de plaies.

2. Utilisation selon la revendication 1, **caractérisée en ce qu'**elle provient des espèces Musca domestica, Calliphora erythrocephala, Sarcophaga carnaria, Phormia regina, Lucilla sericata et/ou Lucilla caesar.

3. Utilisation selon l'une quelconque ou selon les deux revendications 1 et 2**, caractérisée en ce que** l'on homogénéise toutes les chrysalides ou certains des constituants des chrysalides des genres Musca, Calliphora, Phormia, Sarcophaga et/ou Lucilia, par voie mécanique, par addition de produits chimiques et/ou par ultrasons.

4. Utilisation selon la revendication 3, **caractérisée en ce que** les homogénats sont purifiés, par exemple, de manière à libérer ces derniers des constituants non dissous, et de manière à conserver les constituants solubles.

5. Utilisation selon l'une quelconque ou selon plusieurs des revendications 1 à 4, **caractérisée en ce que** l'on effectue la séparation de constituants non solubles par centrifugation, par ultracentrifugation, par séparation de phases ou par filtration, et **en ce que** l'on conserve exclusivement le surnageant.

6. Utilisation selon l'une quelconque ou selon plusieurs des revendications 1 à 5, **caractérisée en ce que** l'on réalise une filtration stérile en utilisant un filtre présentant un diamètre de pores de 0,1 µm à 0,4 µm.

7. Utilisation selon l'une quelconque ou selon plusieurs des revendications 1 à 6, **caractérisée en ce que** l'on ajoute des agents de conservation, par exemple, des substances microbicides ou anti-oxydantes.

8. Utilisation selon l'une quelconque ou selon plusieurs des revendications 1 à 7, **caractérisée en ce que** l'on ajoute des adjuvants appropriés au plan pharmaceutique et acceptables au plan physiologique.
